(19) Europäisches Patentamt
European Patent Office
Office européen des brevets

(11) Veröffentlichungsnummer: **0 041 243**
**B1**

(12) # EUROPÄISCHE PATENTSCHRIFT

(45) Veröffentlichungstag der Patentschrift: **21.12.83**

(51) Int. Cl.³: **C 07 C 103/52,** A 61 K 37/38

(21) Anmeldenummer: **81104103.7**

(22) Anmeldetag: **28.05.81**

(54) **Nonapeptid, Verfahren zu seiner Herstellung, dieses enthaltendes Mittel und seine Verwendung.**

(30) Priorität: **03.06.80 DE 3020941**

(43) Veröffentlichungstag der Anmeldung: **09.12.81 Patentblatt 81/49**

(45) Bekanntmachung des Hinweises auf die Patenterteilung: **21.12.83 Patentblatt 83/51**

(84) Benannte Vertragsstaaten: **AT BE CH DE FR GB IT LI NL SE**

(56) Entgegenhaltungen:
**DD - A - 135 078**
**DE - A - 2 438 350**
**DE - A - 2 446 005**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **König, Wolfgang, Dr., Eppsteiner Strasse 25, D-6238 Hofheim am Taunus (DE)**
Erfinder: **Geiger, Rolf, Dr., Prof., Heinrich-Bleicher-Strasse 33, D-6000 Frankfurt am Main 50 (DE)**
Erfinder: **Sandow, Jürgen Kurt, Dr., Am Haideplacken 22, D-6240 Königstein/Taunus (DE)**

EP 0 041 243 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Nonapeptid, Verfahren zu seiner Herstellung, dieses enthaltendes Mittel und seine Verwendung

Gonadoliberin ist ein Dekapeptid der Struktur

Glu-His-Trp-Ser-Tyr-Gly-Leu-Arg-Pro-Gly-$NH_2$

(Biochem. Biophys. Res. Commun. 43, 1334 (1971)). Es wird im Hypothalamus gebildet und bewirkt in der Hypophyse eine Stimulierung der luteotropen und follikelstimulierenden Hormone.

Derivate dieser Dekapeptide mit stärkerer Wirkung erhält man bekanntermaßen, wenn Glycin in Position 6 durch gewisse D-Aminosäuren oder Glycinamid in Position 10 durch Äthylamin ersetzt wird (J. Med. Chem. 16, 1144 (1973)). Besonders günstig erwiesen sich in Position 6 trifunktionelle D-Aminosäuren, deren dritte Funktion durch Schutzgruppen des tert.-Butyl-Typs blockiert sind (Peptides Chemistry, Structure, Biology, ed. R. Walter and J. Meienhofer, pp. 883–888, Ann Arbor, Michigan 1975). Man fand, daß O-tert.-Butyl-D-serin die stärkste biologische Wirkung hat. Ist die tert.-Butylgruppe, wie beim D-Tert-Leucin sehr nahe am Peptidgerüst, so ist die biologische Aktivität sehr schwach. Wird der Abstand der tert.-Butylgruppe von der Peptidkette größer, wie bei den ω-tert.-Butylestern von D-Asp und D-Glu oder bei ε-tert.-butyloxy-carbonyl-D-lysin, so sinkt die biologische Wirkung im Vergleich zu O-tert.-Butyl-D-serin.

Es wurde gefunden, daß bei einer Substitution des Glycins in Position 6 des Gonadoliberins durch den D-α-Aminoadipinsäure-δ-tert.-butylester, bei dem die tert.-Butylgruppe noch weiter als bei der D-Asparaginsäureverbindung bzw. der aus der DE-A-2 438 350 bekannten D-Glutaminsäureverbindung, aber noch nicht so weit wie bei der D-Lysinverbindung entfernt ist, ein Gonadoliberin-wirksames Peptid mit besonders guter Wirksamkeit erhalten wird.

Gegenstand der Erfindung ist ein Peptid der Formel

Glu-His-Trp-Ser-Tyr-D-Aad($OBu^t$)-Leu-Arg-Pro-NH-$C_2H_5$

worin D-Aad($OBu^t$) für den D-α-Aminoadipinsäure-δ-tert.-butylester steht.

Gegenstand der Erfindung ist weiterhin ein Verfahren zur Herstellung dieses Peptids, das dadurch gekennzeichnet ist, daß man

A) Glu-His-Trp-OH mit H-Ser-Tyr-D-Aad($OBu^t$)-Leu-Arg-Pro-NH-$C_2H_5$,

B) Glu-His-OH oder Glu-His(Dnp)-OH mit H-Trp-Ser-Tyr-D-Aad($OBu^t$)-Leu-Arg-Pro-NH-$C_2H_5$

oder

C) Glu-OH mit H-His-Trp-Ser-Tyr-D-Aad($OBu^t$)-Leu-Arg-Pro-NH-$C_2H_5$

kondensiert, und ggf. die Dnp Schutzgruppen abspaltet.

Ferner ist Gegenstand der Erfindung ein Mittel, enthaltend das genannte Dekapeptid und seine Verwendung zur Ferlititätssteigerung oder Kontrazeption.

Für die Kondensation nach Verfahrensweise A kann man besonders vorteilhaft die Azidmethode verwenden, da die Vorstufe, das

Glu-His-Trp-hydrazid

eine gut herstellbare Substanz ist. Die Kondensation von

Glu-His-Trp-OH

mit dem C-terminalen Hexapeptid kann auch mit Dicyclohexylcarbodiimid in Verbindung mit racemisierungssenkenden Zusätzen wie 1-Hydroxybenzotriazol, N-Hydroxysuccinimid oder 3-Hydroxy-4-oxo-3.4-dihydro-1.2.3-benzotriazin ausgeführt werden.

Bei der Verfahrensweise B kann man die unter A beschriebenen Methoden zur Knüpfung von

Glu-His-OH

mit dem C-terminalen Heptapeptid heranziehen. Besser verläuft die Synthese, wenn man die Imidazolfunktion des Histidins intermediär mit einem 2.4-Dinitrophenylrest schützt und zur Kondensation Dicyclohexylcarbodiimid in Verbindung mit 3-Hydroxy-4-oxo-3.4-dihydro-1.2.3-benzotriazin verwendet. Die Racemisierung des Histidins liegt bei dieser Methode unter 1–2%. Nach der Kupplung wird durch Zusatz von Hydrazin zur Reaktionsmischung die 2.4-Dinitrophenylschutzgruppe

wieder abgespalten.

Bei der Verfahrensweise C empfiehlt sich als bevorzugte Reaktion die Verwendung eines Aktivesters der Pyroglutaminsäure zur Knüpfung, wobei die Umsetzung durch Zusatz von 1-Hydroxybenztriazol katalysiert werden kann. Infrage kommen hierzu vor allem Aktivester vom Phenyltyp wie 2.4.5-Trichlorphenylester oder 4-Nitrophenylester. Die Umsetzung der Pyroglutaminsäure mit dem C-terminalen Oktapeptid ist aber auch mit der Azidmethode, der DCCI-Methode, der gemischten Anhydridmethode oder anderen in der Peptidchemie üblichen Methoden möglich.

Die Synthese der jeweiligen C-terminalen Peptide kann beispielsweise in der in den drei Kondensationsschemata wiedergegebenen Weise erfolgen.

Kondensationsschema A

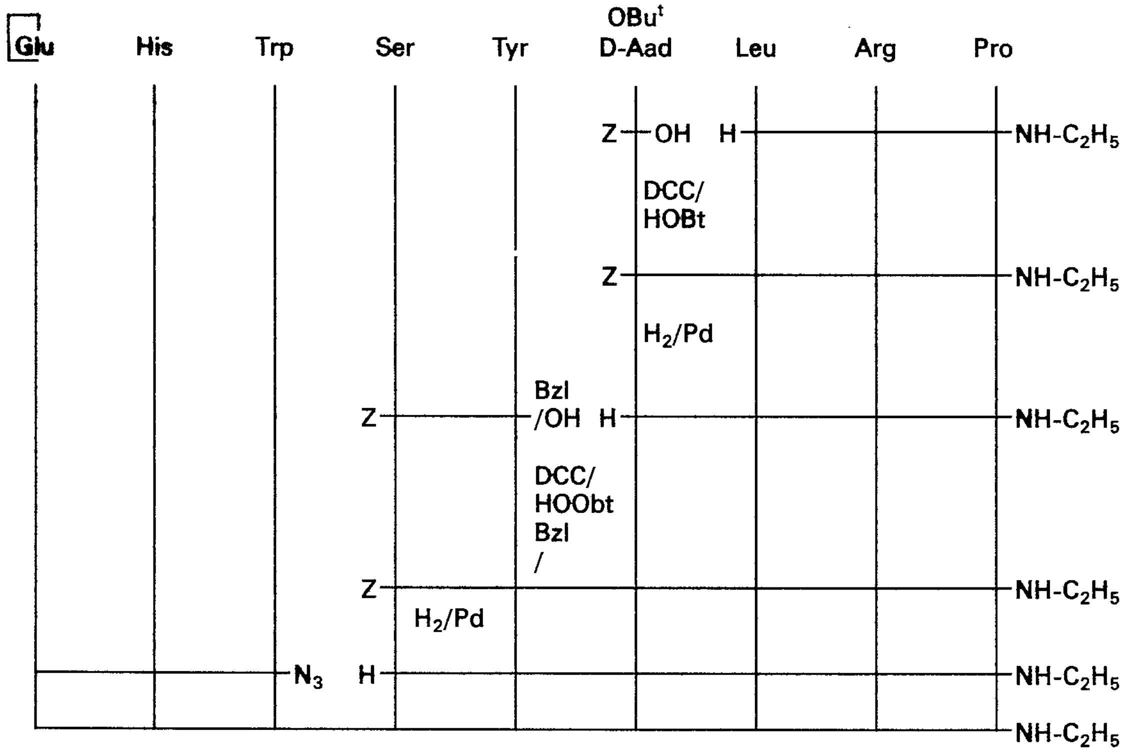


Kondensationsschema B

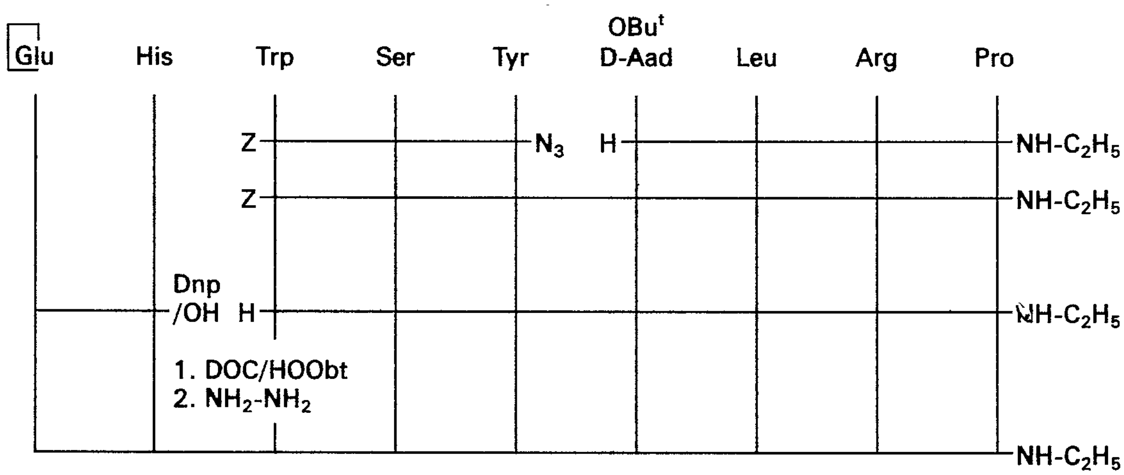


Kondensationsschema C

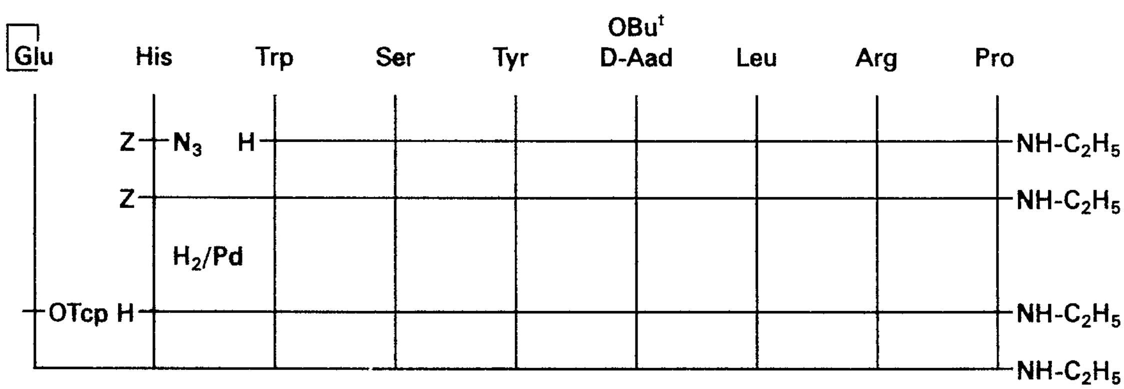

Die D-Aminoadipinsäure kommt in der Natur vor. Eine bevorzugte Quelle ist das Cephalosporin C, in dem sie die Seitenkette bildet. Sie läßt sich bei geeigneter Aufarbeitung bei der Gewinnung der 7-Amino-cephalosporansäure leicht in großen Mengen gewinnen. Demgegenüber ist die chemische Synthese der D-Aminoadipinsäure, insbesondere durch eine verlustreiche Racematspaltung, aufwendiger. Die leichte Zugänglichkeit der in der Natur vorkommenden D-Aminoadipinsäure, bildet neben der guten biologischen Wirkung der Verbindung einen weiteren Vorteil der Erfindung. Der in der Literatur bereits beschriebenen Z-D-Aad-OBzl (Bull. Soc. Chim. Belges 77 (1968), Seiten 587–596) kann mit Isobutylen in Methylenchlorid unter $H_2SO_4$-Katalyse zum entsprechenden δ-tert.-Butylester umgesetzt werden. Eine alkalische Verseifung liefert Z-D-Aad(OBu$^t$)-OH, das entsprechend Kondensationsschema A zur Synthese eingesetzt werden kann.

Das erfindungsgemäße Nonapeptid zeichnet sich durch eine gute Löslichkeit in wäßrigen Pufferlösungen aus. Das macht die Verbindung geeignet für die intranasale Applikation, für die erfahrungsgemäß die Löslichkeit der limitierende Faktor sein kann. Die Löslichkeit ist bedeutend größer als die anderer Gonadoliberinanaloga mit vergleichbarer Wirkungsstärke, insbesondere den D-Trp$^6$- oder D-His(Bzl)$^6$-Analoga.

Das Nonapeptid wirkt bereits in niedriger Dosierung durch Ausschüttung der Gonadotropine fertilitätssteigernd und in wiederholter täglicher hoher Dosierung hemmend auf die Ausschüttung der Gonadotropine und damit kontrazeptiv.

Die bevorzugte Anwendungsform beim Menschen ist die intranasale Applikation, da die Resorption aus dem Magen-Darmtrakt nur gering ist und eine parenterale Anwendung für den Patienten unzweckmäßig ist. Mittels eines Dosierzerstäubers werden über eine Spraydüse etwa 0,1 ml einer Pufferlösung, in der die erforderliche Menge des Wirkstoffs gelöst ist, in die Nase gesprüht.

Indikationen sind z. B. bei der Frau die primäre, aber vorwiegend die secundäre Amenorrhoe, die Corpus luteum Insuffizienz und beim Mann die Oligospermie. Behandelt werden kann ferner die Pubertas tarda beider Geschlechter und der Kryptorchismus bei Knaben.

Die intranasale Verabreichung bewegt sich im allgemeinen bei einer Tagesdosis von 0,025 bis 0,1 mg/Patient, wobei wegen der Langzeitwirkung die Substanz meist nur alle 2 bis 3 Tage appliziert zu werden braucht. Beim Kryptorchismus genügen bereits 0,01 bis 0,025 mg/Tag und Patient.

Für die kontrazeptive Anwendung muß das Nonapeptid täglich intranasal in einer Dosierung von 0,1–0,4 mg, vorzugsweise am Morgen und Abend je 0,1 mg, gegeben werden. Bei parenteraler Applikation können die Dosierungen gegenüber der Intranasal-Dosis um etwa eine Zehnerpotenz gesenkt werden.

In der Veterinärmedizin wird das Nonapeptid vorzugsweise parenteral angewendet. Es kann zur Behandlung von acyclischen Tieren und und zur Ovulationsinduktion und -synchronisation eingesetzt werden. Die Dosis schwankt je nach Tierart.

Empfohlen wird z. B. beim Rind eine Dosis von 0,01–0,02 mg, bei der Stute 0,02–0,04 mg und beim Kaninchen 0,0005–0,0008 mg.

## Beispiel 1

### a) Z-D-Aad(OBu$^t$)-OBzl

Zu einer Lösung von 40 g (ca. 104 mmol) Z-D-Aad-OBzl in 400 ml Methylenchlorid gibt man 400 ml Isobutylen und bei −20° 4 ml konz. Schwefelsäure. Man läßt die Mischung 3 Tage bei Raumtemperatur in einem Autoklaven stehen. Der Ansatz wird mit $NaHCO_3$-Lösung verrührt um die Schwefelsäure zu neutralisieren und mit einem Stickstoffstrom das überschüssige Isobutylen entfernt. Die Methylenchloridphase wird mit Wasser ausgeschüttelt, über $Na_2SO_4$ getrocknet und eingeengt. Es bleiben 46,3 g eines Öls zurück, das über 500 g Kieselgel in einer Mischung aus Methylenchlorid/Aceton (9 : 1) chromatographiert wird. Das Fluat, das Z-Aad(OBu$^t$)-OBzl enthält, wird eingeengt. Ausbeute: 42,35 g eines hellen Öls (92%).

### b) Z-D-Aad(OBu$^t$)-OH · Cyclohexylamin

Zu einer Lösung von 36,7 g (83,1 mmol) Z-D-Aad(OBu$^t$)-OBzl in 175 ml Dioxan gibt man 40 ml Wasser und 87,4 ml 1N NaOH. Man rührt 3 Stunden bei Raumtemperatur, neutralisiert mit etwas $KHSO_4$-Lösung und engt im Vakuum ein. Der Rückstand wird bei Eiskühlung zwischen 300 ml Essigester und 87 ml 1N Schwefelsäure verteilt. Die Esssigesterphase wird einmal mit einer $KHSO_4$-Lösung und zweimal mit Wasser ausgeschüttelt, über $NA_2SO_4$ getrocknet und eingeengt. Das zurücbleibende Öl wird in Äther gelöst. Dazu gibt man 9,45 ml Cyclohexylamin und läßt 4 Stunden im Kühlraum stehen. Der Niederschlag wird abgesaugt, mit Äther gewaschen und getrocknet. Ausbeute: 30,1 g (80%), Schmp. 135°, $[\alpha]_D^{23} = -7{,}1°$ (c = 1, in Methanol).

$C_{24}H_{38}N_2O_6$ (450,59)

| | | | |
|---|---|---|---|
| Ber. | C 63,97 | H 8,50 | N 6,22 |
| Gef. | C 63,6 | H 8,5 | N 6,3 |

c) Z-D-Aad(OBu$^t$)-Leu-Arg-Pro-NH-$C_2H_5$,
$C_{37}H_{60}N_8O_8$ (MG 744.95)

9 g (20 mmol) Z-D-Aad(OBu$^t$)-OH · Cyclohexylamin werden zwischen 40 ml Essigester und 20 ml 1N $H_2SO_4$ verteilt. Die Essigesterphase wird nacheinander mit 10 ml $KHSO_4/K_2SO_4$-Lösung und mit 30 ml Wasser ausgeschüttelt, über $Na_2SO_4$ getrocknet und eingeengt. Der ölige Rückstand wird zusammen mit 15,11 g (20 mmol) H-Leu-Arg-Pro-NH-$C_2H_5$-ditosylat und 2,7 g (20 mmol) 1-Hydroxybenzotriazol (=HOBt) in 50 ml Dimethylformamid gelöst. Man kühlt auf 0°C ab und gibt 5,12 ml (40 mmol) N-Äthylmorpholin und 4,53 g (22 mmol) Dicyclohexylcarbodiimid zu. Man rührt eine Stunde bei 0°C und 6 Stunden bei Raumtemperatur, läßt über Nacht bei Raumtemperatur stehen, saugt vom ausgefallenen Niederschlag ab und verteilt das Filtrat zwischen 250 ml n-Butanol und 250 ml gesättigter NaCl-Lösung. Die n-Butanolphase wird zweimal mit 125 ml gesättigter $NaHCO_3$-Lösung und einmal mit 125 ml Wasser ausgeschüttelt. Die n-Butanolphase wird eingeengt und der Rückstand mit 200 ml Essigsester verrieben. Dazu gibt man noch 200 ml Petroläther und saugt den Niederschlag ab. Ausbeute 15,05 g. Die Rohsubstanz wird aus 110 ml Essigester umkristallisiert.
Ausbeute danach 10,71 g (72%).
Die Substanz ist amorph und beginnt ab 70° C sich zu zersetzen.
$[\alpha]_D^{22} = -41,4°$ (c=1, in Methanol).
Aus der Mutterlauge können durch Zusatz von Petroläther weitere 2,88 g Substanz gefällt werden.
Gesamtausbeute 13,59 g (91%).

d) H-D-Aad(OBu$^t$)-Leu-Arg-Pro-NH-$C_2H_5$ · 2 HCl,
$C_{29}H_{56}Cl_2N_8O_6$ (MG 683.7)

12,5 g (ca. 16,8 mmol) Z-D-Aad(OBu$^t$)-Leu-Arg-Pro-NH-$C_2H_5$ werden in 150 ml Methanol gelöst und bei pH 4,5 unter Zugabe von Pd/$BaSO_4$-Katalysator und unter Zufluß von 2N methanolischer Salzsäure (Autotitrator) katalytisch hydriert. Nach beendigter Hydrierung wird der Katalysator abgesaugt und das Filtrat eingeengt. Der Rückstand wird mit Essigester verrieben, abgesaugt und getrocknet. Ausbeute: 10,85 g (94%) amorphe Substanz. Schmp. 82–85°, $[\alpha]_D^{23} = -48,9°$ (c=l, in 90proz. Essigsäure).

e) Z-Trp-Ser-Tyr-D-Aad(OBu$^t$)-Leu-Arg-Pro-NH-$C_2H_5$,
$C_{60}H_{48}N_{12}O_{13}$, (MG 1181.4)

4,51 g (7,7 mmol) Z-Trp-Ser-Tyr-hydrazid werden in 40 ml einer Dimethylformamid/Dimethylacetamid-Mischung (1 : 1) gelöst. Dazu gibt man bei −30° C 4,31 ml 7N HCl in Dioxan (etwa 30 mmol) und 1,08 ml (9,1 mmol) tert.-Butylnitrit. Man rührt 20 Minuten bei −10° C, gibt 320 mg (4,7 mmol) $NaN_3$ zu, rührt weitere 5 Minuten bei −10° C, kühlt auf −40° C ab, gibt 5,85 ml (45 mmol) N-Äthylmorpholin und 5,13 g (7,5 mmol) H-D-Aad(OBu$^t$)-Leu-Arg-Pro-NH$C_2H_5$ · 2 HCl zu und rührt bei 0°C 6 Stunden. Anschließend läßt man zwei Tage bei 4°C stehen. Von einem auftretenden Niederschlag wird abgesaugt. Das Filtrat wird zwischen 200 ml gesättigter NaCl-Lösung und 150 ml n-Butanol verteilt. Die n-Butanolphase wird zweimal mit 75 ml $KHSO_4$-Lösung und einmal mit 75 ml gesättigter $NaHCO_3$-Lösung ausgeschüttelt und eingeengt. Der Rückstand wird mit 100 ml Essigester verrieben, abgesaugt und getrocknet.
Ausbeute: 7,9 g (89%) amorphe Substanz. $[\alpha]_D^{22} = -27,8°$ (c=l, in 90proz. Essigsäure), Zersetzung ab 138° C.

f) H-Trp-Ser-Tyr-D-Aad(OBu$^t$)-Leu-Arg-Pro-NH-$C_2H_5$ · 2 HCl,
$C_{52}H_{80}Cl_2N_{12}O_{11}$ (MG 1120.22)

7,7 g (etwa 6,5 mmol) Z-Trp-Ser-Tyr-D-Aad(OBu$^t$)-Leu-Arg-Pro-NH-$C_2H_5$ werden analog Beispiel 1 d katalytisch hydriert. Ausbeute: 6,27 g (86%) amorphe Substanz. $[\alpha]_D^{22} = -22,2°$ (c=l, in 90prozentiger Essigsäure).

g) Glu-His-Trp-Ser-Tyr-D-Aad(OBu$^t$)-Leu-Arg-Pro-NH-$C_2H_5$diacetat

Zu einer Lösung von 1,1 g

Glu-His-(Dnp)-OH-hemihydrat

(2,5 mmol), 2,8 g (2,5 mmol) H-Trp-Ser-Tyr-D-Aad(OBu$^t$)-Leu-Arg-Pro-NH-$C_2H_5$ · 2 HCl und 407 mg 3-Hydroxy-4-oxo-3.4-dihydro-1.2.3-benzotriazin in 15 ml Dimethylacetamid gibt man bei 0° C 0,325 ml N-Äthylmorpholin und 550 mg Dicyclohexylcarbodiimid. Man rührt 2 – 3 Stunden bei 0° C und läßt über Nacht bei Raumtemperatur stehen. Der Niederschlag wird abgesaugt. Mit 5 ml Dimethylacetamid wird nachgewaschen. Zum Filtrat gibt man 0,25 ml 100prozentiges Hydrazinhydrat und läßt 4 Stunden bei Raumtemperatur stehen. Danach wird die Substanz mit 140 ml Essigester gefällt und abgesaugt. Der Niederschlag wird in etwa 10 ml Methanol verrieben und erneut mit 140 ml Essigester gefällt. Der Niederschlag wird zwischen 100 ml n-Butanol und 100 ml gesättigter wäßriger $NaHCO_3$-Lösung verteilt. Die n-Butanolphase wird mit 70 ml $NaHCO_3$-Lösung ausgeschüttelt und eingeengt. Der Rückstand wird in ca. 30 – 40 ml verdünnter Essigsäure gelöst und über einen stark basischen Ionenaustauscher in der Acetatform chromatographiert. Das Eluat wird eingeengt und an einem hydroxypropylierten vernetzten Dextrangel in einer Wasser/Essigsäure/n-Butanol-Mischung (10 : 0,8 : 1 Vol.-Teile) chromatographiert.
Ausbeute 1,16 g (33%).
$[\alpha]$ = – 41,6° (c = l, in Methanol)
Die Aminosäureanalyse zeigt die korrekte Aminosäurezusammensetzung und einen Proteingehalt von 85 – 90%. Das UV-Spektrum zeigt das charakteristische Tryptophan-Spektrum und einen Proteingehalt von 87%.

## Beispiel 2

a) Z-His-Trp-Ser-Tyr-D-Aad(OBu$^t$)-Leu-Arg-Pro-NH-$C_2H_5$

Zu einer Lösung von 910 mg (3 mmol) Z-His-hydrazid in 12 ml 1N HCl gibt man bei 0° C 207 mg (3 mmol) $NaNO_2$ in wenig Wasser gelöst hinzu. Nach 5 Minuten bei 0°C gibt man 20 ml kalten Essigester zu und stellt mit kalter gesättigter Sodalösung auf pH 9. Die Essigesterphase wird abgetrennt, die wäßrige Phase noch einmal mit Essigester extrahiert und die vereinigten Essigesterphasen nach Trocknen über $Na_2SO_4$ auf etwa 2 ml eingeengt. Dazu gibt man eine Lösung von 1,12 g (1 mmol) H-Trp-Ser-Tyr-D-Aad(OBu$^t$)-Leu-Arg-Pro-NH-$C_2H_5$ · 2 HCl in ca. 3 ml Dimethylformamid und 0,39 ml (3 mmol N-Äthylmorpholin. Anderntags wird zwischen 50 ml n-Butanol und 50 ml gesättigter $NaHCO_3$-Lösung verteilt. Die n-Butanolphase wird zweimal mit je 25 ml gesättigter $NaHCO_3$-Lösung und einmal mit 25 ml Wasser ausgeschüttelt und eingeengt. Der Rückstand wird mit Äther verrieben, abgesaugt und getrocknet.
Ausbeute: 1,3 g (100%), Schmp. 133 – 135° und Zers. $[\alpha]_D^{22}$ = – 37,9° (c = l, Methanol)

b) H-His-Trp-Ser-D-Aad(OBu$^t$)-Leu-Arg-Pro-NH-$C_2H_5$ · 2 HCl

1,3 g Z-His-Trp-Ser-Tyr-D-Aad(OBu$^t$)-Leu-Arg-Pro-NH-$C_2C_5$ werden in Methanol gelöst und unter Zugabe von 2N methanolischer Salzsäure bei pH 4,5 (Autotitrator) katalytisch (Pd/$BaSO_4$-Katalysator) hydriert. Nach beendigter Reaktion wird der Katalysator abgesaugt und das Filtrat eingeengt. Der Rückstand wird mit Äther verrieben, abgesaugt und getrocknet. Ausbeute 920 mg (73%), Schmp. 146 – 149 u. Z. $[\alpha]_D^{21}$ = – 31,9° (c = l, Methanol).

c) Glu-His-Trp-Ser-Tyr-D-Aad(OBu$^t$)-Leu-Arg-Pro-NH-$C_2H_5$, Acetat

Zu einer Lösung von 628 mg (0,5 mmol) H-His-Trp-Ser-Tyr-D-Aad(OBu$^t$)-Leu-Arg-Pro-NH-$C_2H_5$ · 2 HCl und 67,5 mg 1-Hydroxybenzotriazol in 1 ml Dimethylformamid gibt man 0,125 ml N-Äthylmorpholin und 155 mg

Glu-OTcp

Man läßt einen Tag bei Raumtemperatur stehen, gibt 0,1 ml Hydrazinhydrat zu, rührt 2 Stunden bei Raumtemperatur und verteilt zwischen 10 ml n-Butanol und 10 ml gesättigter $NaHCO_3$-Lösung. Die n-Butanolphase wird eingeengt und mit Äther verrieben, abgesaugt und getrocknet. Die so gewonnene Rohsubstanz wird in verdünnter Essigsäure gelöst und analog Beispiel 1 g an einem stark basischen Ionenaustauscher und an einem hydroxypropylierten vernetzten Dextrangel chromatographiert. Ausbeute 180 mg (25%). DC: entspricht dem im Beispiel 1 g gewonnenen Material (auf Kieselgel G in Chloroform/Methanol/Eisessig/Wasser (60 : 45 : 6 : 14 Vol-Teile)).

Beispiel 3

a) Z-Ser-Tyr(Bzl)-D-Aad($OBu^t$)-Leu-Arg-Pro-NH-$C_2H_5$

Zu einer Lösung von 1 g (ca. 2 mmol) Z-Ser-Tyr(Bzl)-OH, 1,4 g (2 mmol) H-D-Aad($OBu^t$)-Leu-Arg-Pro-NH-$C_2H_5$ · 2 HCl und 0,326 g (2 mmol) 3-Hydroxy-4-oxo-3.4-dihydro-1.2.3-benzotriazin in 8 ml Dimethylacetamid gibt man bei 0°C 0,26 ml N-Äthylmorpholin und 440 mg Dicyclohexylcarbodiimid. Man rührt zwei Stunden bei 0°C und läßt anschließend bei Raumtemperatur über Nacht stehen. Der Niederschlag wird abgesaugt und das Filtrat zwischen 50 ml n-Butanol und 50 ml gesättigter NaCl-Lösung verteilt. Die n-Butanolphase wird zweimal mit je 25 ml gesättigter NaHCO-Lösung und einmal mit 25 ml Wasser ausgeschüttet und eingeengt. Der Rückstand wird mit Äther verrieben, abgesaugt und getrocknet.
Ausbeute 2,05 g (94%), amorph $[\alpha]_D^{22} = -37,4°$ (c=l, Methanol)

b) H-Ser-Tyr-D-Aad($OBu^t$)-Leu-Arg-Pro-NH-$C_2H_5$ · 2 HCl

1,9 g (1,75 mmol Z-Ser-Tyr(Bzl)-D-Aad($OBu^t$)-Leu-Arg-Pro-NH-$C_2H_5$ werden in 150 ml Methanol gelöst und analog Beispiel 1d katalytisch hydriert. Der Rückstand wird mit Äther verrieben, abgesaugt und getrocknet.
Ausbeute 1,33 g (83%), Schmp. 134–136°C $[\alpha]_D^{22} = -38,4°$ (c=l, Methanol).

c) Glu-His-Trp-Ser-Tyr-D-Aad($OBu^t$)-Leu-Arg-Pro-NH-$C_2H_5$, Acetat

Zu einer Lösung von 500 mg

Glu-His-Trp-NH-$NH_2$

in 6 ml Dimethylformamid gibt man bei −30°C 0,66 ml einer 6,05 N HCl/Dioxan-Lösung und 1,2 ml einer 10prozentigen tert.-Butylnitrit-Lösung in absolutem Dioxan. Man rührt 20 Minuten bei −10°C und gibt bei −40°C 918 mg rohes H-Ser-Tyr-D-Aad($Bu^t$)-Leu-Arg-Pro-NH-$C_2H_5$ · 2 HCl und 0,78 ml N-Äthylmorpholin zu. Man läßt über Nacht bei 4°C stehen, engt ein und verreibt den Rückstand mit Äther. Die Substanz wird in Wasser gelöst und analog Beispiel 1g an einem stark basischen Ionenaustauscher und anschließend an einem hydroxypropylierten vernetzten Dextrangel chromatographiert.
Ausbeute 410 mg (31%),
DC: Identisch mit der Substanz, die in Beispiel 1g gewonnen wurde.

**Patentansprüche:**
**Für die Vertragsstaaten: BE, CH, DE, FR, GB, IT, Li, NL, SE**

1. Peptid der Formel

Glu-His-Trp-Ser-Tyr-D-Aad($OBu^t$)-Leu-Arg-Pro-NH-$C_2H_5$ in der -D-Aad($OBu^t$) für den D-$\alpha$-Aminoadipinsäure-$\delta$-tert.-Butylester steht.

2. Verfahren zur Herstellung des Peptids nach Anspruch 1, dadurch gekennzeichnet, daß man

a) Glu-His-Trp-OH mit H-Ser-Tyr-D-Aad($OBu^t$)-Leu-Arg-Pro-NH-$C_2H_5$

oder

b) Glu-His-OH oder Glu-His(Dnp)-OH mit H-Trp-Ser-Tyr-D-Aad($OBu^t$)-Leu-Arg-Pro-NH-$C_2H_5$

oder

c) Glu-OH mit H-His-Trp-Ser-Tyr-D-Aad($OBu^t$)-Leu-Arg-Pro-NH-$C_2H_5$

kondensiert, und gegebenenfalls die Dnp-Schutzgruppe abspaltet.

3. Mittel, enthaltend das Peptid gemäß Anspruch 1, gelöst in einer wäßrigen Pufferlösung.

4. Verwendung des Peptids gemäß Anspruch 1 oder des Mittels gemäß Anspruch 3 zur Fertilitätssteigerung oder Kontrazeption bei Tieren.

5. Peptid gemäß Anspruch 1 zur Verwendung als Mittel zur Fertilitätssteigerung oder Kontrazeption.

6. Mittel gemäß Anspruch 3 zur Verwendung als fertilitätssteigerndes oder kontrazeptives Mittel.

**Patentansprüche für den Vertragsstaat: AT**

1. Verfahren zur Herstellung des Peptids der Formel

Glu-His-Trp-Ser-Tyr-D-Aad(OBu$^t$)-Leu-Arg-Pro-NH-$C_2H_5$ in der -D-Aad(OBu$^t$) für den D-$\alpha$-Aminoadipinsäure-$\delta$-tert.-Butylester steht, daß man

a) Glu-His-Trp-OH mit H-Ser-Tyr-D-Aad(OBu$^t$)-Leu-Arg-Pro-NH-$C_2H_5$

oder

b) Glu-His-OH oder Glu-His(Dnp)-OH mit H-Trp-Ser-Tyr-D-Aad(OBu$^t$)-Leu-Arg-Pro-NH-$C_2H_5$

oder

c) Glu-OH mit H-His-Trp-Ser-Tyr-D-Aad(OBu$^t$)-Leu-Arg-Pro-NH-$C_2H_5$

kondensiert, und gegebenenfalls die Dnp-Schutzgruppe abspaltet.

2. Verfahren gemäß Anspruch 1 zur Herstellung eines Peptids der in Anspruch 1 genannten Formel zur Verwendung als Mittel zur Fetilitätssteigerung oder Kontrazeption.

3. Verfahren zur Herstellung eines Mittels enthaltend ein Peptid gemäß Anspruch 1, dadurch gekennzeichnet, daß dieses Peptid in einer wäßrigen Pufferlösung aufgelöst wird.

**Claims for the Contracting State: AT**

1. Process for the preparation of the peptide of the formula

Glu-His-Trp-Ser-Tyr-D-Aad(OBu$^t$)-Leu-Arg-Pro-NH-$C_2H_5$ in which -D-Aad(OBu$^t$) represents D-$\alpha$-aminoadipic acid $\delta$-tert.-butyl ester, which comprises:

a) subjecting Glu-His-Trp-OH to a condensation reaction with H-Ser-Tyr-D-Aad(OBu$^t$)-Leu-Arg-Pro-NH-$C_2H_5$, or

b) subjecting Glu-His-OH or Glu-His(Dnp)-OH to a condensation reaction with H-Trp-Ser-Tyr-D-Aad(OBu$^t$)-Leu-Arg-Pro-NH-$C_2H_5$, or

c) subjecting Glu-OH to a condensation reaction with H-His-Trp-Ser-Tyr-D-Aad(OBu$^t$)-Leu-Arg-Pro-NH-$C_2H_5$,

and, if appropriate, splitting off the Dnp protective group.

2. Process as claimed in claim 1 for preparing a peptide of the formula as designated in claim 1 for use as agent for increasing fertility or for contraception.

3. Process for preparing an agent containing the peptide as claimed in claim 1, characterized in that this peptide is dissolved in an aqueous buffer solution.

**Claims for the Contracting States: BE, CH, DE, FR, GB, IT, LI, NL, SE**

1. Peptide of the formula

Glu-His-Trp-Ser-Tyr-D-Aad(OBu$^t$)-Leu-Arg-Pro-NH-$C_2H_5$ in which -D-Aad(OBu$^t$) represents D-$\alpha$-aminoadipic acid $\delta$-tert.-butyl ester.

2. Process for the preparation of the peptide as claimed in claim 1, which comprises:

a) subjecting Glu-His-Trp-OH to a condensation reaction with H-Ser-Tyr-D-Aad(OBu$^t$)-Leu-Arg-Pro-NH-$C_2H_5$, or

b) subjecting Glu-His-OH or Glu-His(Dnp)-OH to a condensation reaction with H-Trp-Ser-Tyr-D-Aad(OBu$^t$)-Leu-Arg-Pro-NH-$C_2H_5$, or

c) subjecting Glu-OH to a condensation reaction with H-His-Trp-Ser-Tyr-D-Aad(OBu$^t$)-Leu-Arg-Pro-NH-$C_2H_5$,

and, if appropriate, splitting off the the Dnp protective group.

3. Agent containing the peptide as claimed in claim 1, dissolved in an aqueous buffer solution.

4. Use of the peptide as claimed in claim 1 or of the agent as claimed in claim 3 for increasing fertility or for contraception in animals.

5. Peptide as claimed in claim 1 for use as agent foı increasing fertility or for contraception.

6. Agent as claimed in claim 3 for use as agent for increasing fertility or for contraception.

**Revendications pour l'Etat contractant: AT**

1. Procédé de préparation du peptide de formule

Glu-His-Trp-Ser-Tyr-D-Aad(OBu$^t$)-Leu-Arg-Pro-NH-$C_2H_5$ dans laquelle -D-Aad(OBu$^t$) désigne l'ester $\delta$-tert-butylique de l'acide D-$\alpha$-aminoadipique, caractérisé en ce qu'on condense

a) Glu-His-Trp-OH avec H-Ser-Tyr-D-Aad(OBu$^t$)-Leu-Arg-Pro-NH-$C_2H_5$

ou

b) Glu-His-OH ou Glu-His(Dnp)-OH avec H-Trp-Ser-Tyr-D-Aad(OBu$^t$)-Leu-Arg-Pro-NH-$C_2H_5$

ou

c) Glu-OH avec H-His-Trp-Ser-Tyr-D-Aad(OBu$^t$)-Leu-Arg-Pro-NH-$C_2H_5$

et on élimine le cas échéant le groupe protecteur Dnp.

2. Procédé suivant la revendication 1 pour la préparation d'un peptide répondant à la formule indiquée á la revendication 1 en vue de l'utilisation comme agent pour l'augmentation de la fertilité ou la contraception.

3. Procédé de préparation d'un agent contenant un peptide suivant la revendication 1, caractérisé en ce qu'on dissout ce peptide dans une solution tampon aqueuse.

**Revendications pour les Etats contractants:**
**BE, CH, DE, FR, GB, IT, Li, NL, SE**

1. Peptide de formule

Glu-His-Trp-Ser-Tyr-D-Aad(OBu$^t$)-Leu-Arg-Pro-NH-$C_2H_5$ dans laquelle -D-Aad(OBu$^t$) désigne l'ester $\delta$-tert-butylique de l'acide D-$\alpha$-aminoadipique.

2. Procédé de préparation du peptide suivant la revendication 1, caractérisé en ce qu'on condense

a) Glu-His-Trp-OH avec H-Ser-Tyr-D-Aad(OBu$^t$)-Leu-Arg-Pro-NH-$C_2H_5$

ou

b) Glu-His-OH ou Glu-His(Dnp)-OH avec H-Trp-Ser-Tyr-D-Aad(OBu$^t$)-Leu-Arg-Pro-NH-$C_2H_5$

ou

c) Glu-OH avec H-His-Trp-Ser-Tyr-D-Aad(OBu$^t$)-Leu-Arg-Pro-NH-$C_2H_5$

et on élimine le cas échéant le groupe protecteur Dnp.

3. Agent contenant le peptide suivant la revendication 1 dissous dans une solution tampon aqueuse.

4. Utilisation du peptide suivant la revendication 1 ou de l'agent suivant la revendication 3 pour l'augmentation de la fertilité ou la contraception chez les animaux.

5. Peptide suivant la revendication 1 pour l'utilisation comme agent pour l'augmentation de la fertilité ou la contraception.

6. Agent suivant la revendication 3 pour l'utilisation comme agent augmentant la fertilité ou comme agent contraceptif.